Europäisches Patentamt

(19) European Patent Office

Office européen des brevets

(11) Publication number : **0 320 002 B1**

(12) **EUROPEAN PATENT SPECIFICATION**

(45) Date of publication of patent specification :
23.10.91 Bulletin 91/43

(51) Int. Cl.$^5$ : **A61K 31/44**, A61K 9/72

(21) Application number : **88120632.0**

(22) Date of filing : **09.12.88**

(54) Inhalant.

(30) Priority : **10.12.87 JP 312594/87**

(43) Date of publication of application :
**14.06.89 Bulletin 89/24**

(45) Publication of the grant of the patent :
**23.10.91 Bulletin 91/43**

(84) Designated Contracting States :
**BE CH DE ES FR GB IT LI NL SE**

(56) References cited :
**EP-A- 0 156 243**
**EP-A- 0 215 438**

(73) Proprietor : **KYORIN SEIYAKU KABUSHIKI KAISHA**
**2-5, Kanda Surugadai**
**Chiyoda-ku Tokyo (JP)**

(72) Inventor : **Kamijo, Shinji**
**5-38-10, Soshigaya**
**Setagaya-ku Tokyo (JP)**
Inventor : **Imai, Jun**
**1400-70, Kosugaya-cho Sakae-ku**
**Yokohama-shi Kanagawa-ken (JP)**

(74) Representative : **Vossius & Partner**
**Siebertstrasse 4 P.O. Box 86 07 67**
**W-8000 München 86 (DE)**

Jouve, 18, rue Saint-Denis, 75001 PARIS

## Description

This invention relates to an inhalant containing 3-isobutyryl-2-isopropylpyrazolo[1,5-a]pyridine (hereinafter referred to as ibudilast according to its international nonproprietary name) which is a drug for treating bronchial asthma and cerebrovascular disorder.

Ibudilast is well known as a chemical compound developed by Irikura et al. (JP-B-52-29318 corresponding to US-A-3,850,941 and GB-A-1,378,375) and has been proved by clinical tests to be useful for preventing and treating bronchial asthma. Furthermore its utility as an improved drug for treating cerebrovascular disorder has been reported. When ibudilast is orally administered to a patient in a dosage form having no sustained release, a rapid absorption from the alimentary tract will produce a steep rise of serum concentration, resulting in side-effects such as nausea and vomiting. Therefore, as far as oral drugs are concerned, sustained release capsules and tablets are proposed for the compound (Japanese Laid-Open Patent Application JP-A-60-193913 corresponding to EP-A-0156243). Also the compound has been successfully prepared for rectal administration because of finding out bases for preventing a rapid increase in the serum concentration (JP-A-60-193913).

However, in case of an asthmatic attack, where an immediate effect is needed, a drug form is wanted in place of a sustained release form. Thus, the development of a novel dosage form of ibudilast which assures immediate effects but has no side-effects is desired.

The object of the present invention is to provide drug forms which are easily used and excellent in an immediate effect for bronchial asthma patients. This object has been achieved by the finding that ibudilast is highly effective when it is inhaled via bronchia. This led to the development of a pharmaceutical composition suitable for inhalation (hereinafter called "inhalant") containing ibudilast as active ingredient. The pharmaceutical inhalant containing ibudilast as active ingredient according to this invention may be prepared in the form of a capsule filled with the mixture of pulverized ibudilast and vehicles, and said capsule may be administered by means of a spinhaler® to allow the active ingredient to be inhaled into the lungs.

Also, an aqueous solution of ibudilast or a liquid preparation of ibudilast obtained by means of a solubilizing agent may be employed as an inhalant by the aid of an inhaler, or the solution or liquid may be used as inhalant spray by charging it in a vessel of a nebulizer together with a propellant.

Ibudilast is slightly bitter, however, this may be masked by microencapsulization with a polymeric material.

As for microencapsulizing methods, a coacervation method using gelatin-gum arabic, a solution method, an interfacial polymerization method and a vapor phase method may be employed. The range of particle diameter of microencapsulized ibudilast is preferably 5 to 10 μm.

As for the coacervation, a method of microencapsulating ibudilast with gelatin may be illustrated, wherein pulverized ibudilast is suspended in a solution of gelatin, and to the suspension is added sodium sulfate to cause coacervation and the resultant coacervate is removed and dried to produce the microencapsulated ibudilast. Also, another microencapsulation method is possible, wherein microencapsules are produced by means of addition of gum arabic to gelatin.

As for the solution method, one illustration is that pulverized ibudilast is suspended in a solution of sodium alginate and to the resultant suspension is added calcium chloride to give solidification and then the resultant solid is removed and dried to produce the microencapsulated ibudilast.

As for the vapor phase method, one illustration is that pulverized ibudilast is dispersed in an aqueous solution of gum arabic, gelatin, polyvinylpyrrolidone, methylcellulose, hydroxypropylcellulose or polyvinyl alcohol and the resultant dispersion is dried by means of a spray dryer having a nozzle or a rotating disc to form microencapsulated ibudilast. Also, a liquid dispersion of ibudilast dispersed in a polymeric material may be heated to about 60°C to dissolve ibudilast, and the resultant emulsion may be dried by spraying.

In order to prepare an inhalant of microencapsulated ibudilast, a method may be illustrated, wherein microencapsulated ibudilast is charged into a hard gelatin capsule, and the capsule is mounted in a spinhaler® for inhalation. Also an inhalant made from a solution of ibudilast may be prepared.

As the solubility of ibudilast in water is 0.015%, when a higher concentration of ibudilast is desired, it is preferable to use a solubilizing agent. As far as solubilizing agent is concerned, polyoxyethylene hydrogenated castor oil (HCO-60®), polyoxyethylene sorbitan monooleate (Tween 80®), benzethonium chloride (Triton-X-100®), Lauromacrogol, sodium lauryl sulfate and alcohol may be employed.

An aqueous solution of ibudilast may be prepared by dissolving into a 0.2 to 5% aqueous solution of a solubilizing agent a 0.05 to 0.5% equivalent of ibudilast.

This inhalant may be inhaled, at need, by putting this solution of solubilized ibudilast into a nebulizer.

An inhalant in aerosol dosage form, which is prepared by charging the solution into the vessel of a nebulizer together with a propellant, may be used by inhalation at need.

An inhalant in a nebulizing dosage form may be prepared by mixing the solution with a colorless, non-odorous, chemically stable and non-irritable gas, for example, liquefied petroleum gases such as propane or n-butane, or fluorene gases as well as liquefied fluorocarbons (fulon). Also, as for compressed gases, nitrogen gas, carbonic acid gas and liquefied nitrogen gas are employed.

As a nebulizing container, a container having an actuator for spraying the solution and being effective to exhibit aerosol-function, should be employed.

The inhalant of ibudilast obtained according to this invention, which is excellent in its absorption property, may be employed for bronchial asthma patients with great convenience and permits a rapid and easy administration of a definite amount of drug to a desired region. Moreover, the administration of the inhalant causes no physical irritation and is painless. A local dosage of said aerosol requires no washing-off of the drug as required in the case of a ointment and hence is very convenient in handling.

The following examples illustrate the preferred embodiments of this invention, but are not intended to limit its scope.

Example 1 :

10 g of pulverized ibudilast (average particle diameter : 6 μm) and 40 g of powdered sugar were mixed and a hard gelatin capsule was filled with the mixture to make an inhalant containing 10 mg equivalent of ibudilast per capsule. The capsule may be utilized by setting it to a spinhaler®.

Example 2 :

3 g of gelatin was dissolved in 100 ml of purified water. In the resultant solution 10 g of pulverized ibudilast was suspended and the pH value was adjusted to 4.5. Separately prepared 100 ml of a 3% gum arabic solution was added to the above suspension to produce coacervate, which was removed, filtered, and dried at about 40°C to obtain microencapsulated ibudilast. This microencapsulated ibudilast may be used in the form of hard gelatin capsule filled with, for example, 10 mg equivalent of ibudilast per said capsule.

Example 3 :

25 g of gelatin and 25 g of gum arabic were dissolved in 500 ml of purified water and to the resultant solution was added 100 g of pulverized ibudilast. Then the mixture was agitated, heated to 60°C and emulsified by means of a mixer. Then the emulsion was cooled to room temperature under stirring. This solution was dried by a spray dryer equipped with nozzle and then microencapsulated ibudilast powder was obtained. This powder may be employed in the form of a hard gelatin capsule filled with 10 mg equivalent of ibudilast.

Example 4 :

0.5 g of ibudilast, 10 g of ethanol and 10 g of water were mixed and dissolved. Then a spraying pressure vessel was filled up with resulting solution and 79.5 g of dichlorodifluoromethane. Thus obtained inhalant had an excellent absorptive action when inhaling by spraying this solvent from said vessel.

Example 5 :

0.2 g of ibudilast, 2 g of HCO-60® and 378 g of water were mixed together to make a solution. Then, to this solution were added 60 g of dichlorodifluoromethane, and were charged in a spraying pressure vessel according to a conventional method under pressure to obtain an inhalant.

Example 6 :

0.1 g of ibudilast was dissolved in 700 ml of purified water and the solution was charged in an ampoule or in a vessel made of glass to obtain 0.14 mg/ml of inhalant. This solution may be inhaled at need by putting a definite amount thereof in an inhaling vessel.

As illustrated above, the inhalant containing ibudilast according to this invention as medically active ingredient may provide a rapid action and has an effect which enhances its medical utility.

## Claims

1. An inhalant comprising 3-isobutyryl-2-isopropylpyrazolo[1,5-a]pyridine as active ingredient.

2. An inhalant according to claim 1, wherein the inhalant is in the form of a capsule.

3. An inhalant according to claim 1, wherein the inhalant is in the form of a liquid preparation.

4. An inhalant according to claim 1, wherein the inhalant is in the form of an aerosol.

5. A method for preparing an inhalant which comprises combining 3-isobutyryl-2-isopropylpyrazolo[1,5-a]pyridine as active ingredient with a vehicle which is suitable for inhalation and formulating the combination obtained into a preparation suitable for inhalation.

## Patentansprüche

1. Inhalationsmittel umfassend 3-Isobutyryl-2-isopropyl-pyrazolo[1,5-a]pyridin als Wirkstoff.

2. Inhalationsmittel nach Anspruch 1, wobei das Inhalationmittel in Form einer Kapsel vorliegt.

3. Inhalationsmittel nach Anspruch 1, wobei das Inhalationsmittel in Form einer Flüssigzubereitung vorliegt.

4. Inhalationsmittel nach Anspruch 1, wobei das Inhalationsmittel in Form eines Aerosols vorliegt.

5. Verfahren zur Herstellung eines Inhalationsmittels umfassend die Kombination von 3-Isobutyryl-2-isopropylpyrazolo[1,5-a]pyridin als Wirkstoff mit einem zum Inhalieren geeigneten Träger und das Überführen der erhaltenen Kombination in eine zum Inhalieren geeignete Zubereitung.

**Revendications**

1. Produit pour inhalation comprenant de la 3-isobutyryl-2-isopropyl-pyrazolo[1,5-a]pyridine comme ingrédient actif.

2. Produit pour inhalation selon la revendication 1, dans lequel le produit pour inhalation est sous la forme d'une capsule.

3. Produit pour inhalation selon la revendication 1, dans lequel le produit pour inhalation est sous la forme d'une préparation liquide.

4. Produit pour inhalation selon la revendication 1, dans lequel le produit pour inhalation est sous la forme d'un aérosol.

5. Procédé de préparation d'un produit pour inhalation qui comprend le fait d'associer la 3-isobutyryl-2-isopropyl-pyrazolo[1,5-a]pyridine comme ingrédient actif à un véhicule convenant pour l'inhalation, et de formuler l'association obtenue sous forme d'une préparation convenant pour l'inhalation.